## Europäisches Patentamt
## European Patent Office
## Office européen des brevets

(19)

(11) Veröffentlichungsnummer: **0 093 920**
**B1**

(12) **EUROPÄISCHE PATENTSCHRIFT**

(45) Veröffentlichungstag der Patentschrift:
**18.03.87**

(51) Int. Cl.⁴: **C 12 Q 1/22**

(21) Anmeldenummer: **83103955.7**

(22) Anmeldetag: **22.04.83**

(54) **Bioindikator.**

(30) Priorität: **06.05.82 DE 3217002**

(43) Veröffentlichungstag der Anmeldung:
**16.11.83 Patentblatt 83/46**

(45) Bekanntmachung des Hinweises auf die Patenterteilung:
**18.03.87 Patentblatt 87/12**

(84) Benannte Vertragsstaaten:
**BE CH DE FR GB IT LI NL**

(56) Entgegenhaltungen:
**DE-A-1 492 498**
**US-A-3 440 144**
**US-A-4 291 122**

(73) Patentinhaber: **Merck Patent Gesellschaft mit beschränkter Haftung, Frankfurter Strasse 250, D-6100 Darmstadt (DE)**

(72) Erfinder: **Metz, Harald, Dr., Am Landbach 8, D-6101 Bickenbach (DE)**
Erfinder: **Kappner, Manfred, Dr., Georg- Büchner-Strasse 26, D-6107 Reinheim 3 (DE)**
Erfinder: **Gunkel, Werner, Wingertsweg 33, D-6101 Rossdorf 2 (DE)**

EP 0 093 920 B1

## Beschreibung

Die Erfindung betrifft Bioindikatoren zur mikrobiologischen Kontrolle von Gassterilisationsverfahren.

Bei den Bioindikatoren handelt es sich um Testsysteme, die lebende Mikroorganismen als Indikatoren enthalten. Der Bioindikator wird zusammen mit dem zu sterilisierenden Gut den Sterilisationsbedingungen ausgesetzt. Nach abschluß des Sterilisiervorganges erfolgt eine Kontrolle der Abtötung der Indikator-Mikroorganismen.

Mikrobiologische Sterilisationsindikatoren werden in zunehmendem Maße bei der Überwachung von Sterilisationsverfahren verwendet. Während bei der Kontrolle von Sterilisationsprozessen das Schwergewicht früher auf der Sterilitätsprüfung des sterilisierten Gutes lag, kann heute der Umfang der Sterilitätsprüfung verringert werden, wenn das Sterilisationsverfahren mittels mikrobiologischer Indikatoren kontrolliert und eine ausreichende Sterilisationssicherheit nachgewiesen wird.

Gassterilisationsverfahren beruhen auf der Verwendung von gasförmigen, bakterizid und sporizid wirkenden chemischen Agenzien wie beispielsweise Ethylenoxid und Formaldehyd. Die Abtötungswirkung von chemischen Agenzien wird im wesentlichen durch deren Konzentration, Einwirkungszeit und Temperatur sowie durch die Art und den Zustand der Mikroorganismen bestimmt.

Als quantitatives Maß für die Widerstandsfähigkeit von Mikroorganismen gegenüber Sterilisationsbedingungen dienen die "Dezimale Abtötungszeit" (D-Wert) sowie die "Abtötungszeit". Die "Dezimale Abtötungszeit" gibt die Zeit in Minuten an, innerhalb der die Zahl der vermehrungsfähigen Keime auf 1/10 abnimmt. Die "Abtötungszeit" gibt an, wie lange die Sterilisationsbedingungen andauern müssen, bis am kontaminierten Objekt oder am Bioindikator keine überlebensfähigen Mikroorganismen mehr nachgewiesen werden können.

Die Abtötungszeit der Bioindikator-Testkeime sollte mindestens so lang sein wie die Abtötungszeit der Keime auf dem kontaminierten Probenmaterial, d.h. die Resistenz der Mikroorganismen des Bioindikators soll der Resistenz der Keime am kontaminierten Probenmaterial ähnlich sein.

Die Verwendung von Bioindikatoren zur Kontrolle von Gassterilisationsverfahren ist bekannt. Der wichtigste natürliche Bioindikator ist Sporenerde; dabei handelt es sich meist um Komposterde, die zu 0,2 - 2,0 g in speziellen Päckchen abgepackt ist. Nach der Exposition in der Sterilisationskammer werden die Sporenerde-Päckchen unter aseptischen Bedingungen geöffnet, wonach die Sporenerde in Kulturröhrchen mit einem Nährmedium übergeführt wird. Die Kulturen werden bebrütet und z.B. nach 1, 3, 7 und 14 Tagen auf Trübung infolge Mikroorganismenwachstum überprüft. Trübe Röhrchen werden oft zusätzlich durch Überimpfen einer Probe auf einen Nährboden untersucht.

Die Verwendung von Sporenerde als Bioindikator ist nicht unproblematisch, da die Zusammensetzung der Mikroorganismenflora der Erde und die Resistenz der Mikroorganismen gegenüber den Sterilisationsbedingungen sehr unterschiedlich sein kann. Die Beurteilung der Sterilisationseffizienz ist umständlich und langwierig, da die Proben mehrere Tage bebrütet werden müssen. Oft kann erst nach überimpfung einer Probe auf einen Nährboden eine sichere Aussage über ein Mikroorganismenwachstum gemacht werden, da die Sporenerde selbst bereits zu einer Trübung des Nährmediums führt.

Neben Bioindikatoren mit natürlicher Keimpopulation wie Sporenerde sind auch Bioindikatoren in Gebrauch, die Reinkulturen von bestimmten Mikroorganismen enthalten. Der geeignetste Testorganismus für die Ethylenoxid-Sterilisation ist Bacillus subtilis var. niger in Form seiner Spore. Die Sporen besitzen eine recht große Resistenz gegenüber Ethylenoxid, der Mikroorganismus ist apothogen und dank seiner orangen Pigmentbildung z.B. auf Caseinpepton-Sojamehlpepton-Agar leicht zu erkennen. Bioindikatoren, die Sporen von Reinkulturen der Indikatororganismen enthalten, sind in den unterschiedlichsten Ausführungsformen bekannt, beispielsweise auf Trägermaterialien wie Papier, Kunststoff, Glas, Sand.

Bei den sogenannten Sporenstreifen handelt es sich um Filterpapierstreifen, die mit einer definierten Sporenzahl beladen sind. Sporenstreifen werden gewöhnlich mit einer Sporenkonzentration von etwa $10^6$ Sporen pro Streifen verwendet. Diese Streifen sind einzeln in kleine kuvertartige Verpackungen aus gas- und wasserdampfdurchlässigem Material verpackt. Dieser Bioindikator wird mit dem zu sterilisierenden Gut in die Sterilisationskammer gegeben. Zur Überprüfung der Effizienz des Sterilisationsverfahrens wird der Sporenstreifen nach der Sterilisation mit einer sterilen Pinzette aus der kuvertartigen verpackung entnommen und in ein Kulturröhrchen mit sterilem Nährmedium gegeben. Die Kulturröhrchen werden bebrütet. Das Wachstum überlebender Keime wird duch Trübung des Nährmediums und/oder durch Farbumschlag eines pH-Indikators angezeigt. Die Ablesung der Kulturröhrchen erfolgt meist nach 1, 2, 3, 4 und 7-tägiger Bebrütung. Bei der Entnahme des Sporenstreifens aus der kuvertartigen Verpackung zum Test auf lebensfähige Keime besteht die Gefahr, daß der Sporenstreifem mit lebenden Mikroorganismen kontaminiert wird und somit ein falsches Ergebnis erzielt wird.

Neben Bioindikatoren, bei denen die Sporenträger nach der Sterilisation in ein separates Nährmedium übertragen werden müssen, sind auch komplette Bioindikatorsätze bekannt, die Sporenträger und Nährmedium enthalten Diese Bioindikatoren bestehen aus Kunststoffröhrchen, die einen mit einer definierten Sporenzahl belegten Papierstreifen und eine dünnwandige Glasampulle mit Nährmedium enthalten. Das Kunststoffröhrchen ist mit einer gasdurchlässigen Kappe verschlossen. Zur Überprüfung auf lebensfähige Keime wird durch Zusammendrückan des Kunststofftöhrchens die Glasampulle zerbrochen, wodurch das Nährmedium mit dem Sporenträger in Kontakt tritt. Anschließend wird der Bioindikator bebrütet. Ein Wachstum von Keimen, die die Sterilisation überlebt haben, ist an einem Farbumschlag des pH-Indikators im Kulturmedium zu erkennen. Die Ablesung erfolgt nach 24 und 48 Stunden (US 3,440,144). bei einem weiteren bekannten Bioindikator, der außerhalb der Glasampulle noch feste Kohlenwasserstoffe zur Vermeidung falsch positiver Ergebnisse enthält, erfolgt die Ablesung erst nach 7 Tagen (US 4,291,122).

Die Aufbewahrung des Nährmediums in einer Glasampulle gewährleistet zwar eine wasserdampfdichte Verpackung des Nährmediums, jedoch ist eine Verletzungsgefahr durch Glassplitter beim Zerbrechem der Ampulle nicht auszuschließen.

Die bekannten Bioindikatoren sind oft kostspielig in der Produktion, die Standardisierung der Empfindlichkeit der Indikator-Mikroorganismen gegenüber den Sterilisationsbedingungen ist schwierig, die Handhabung unsicher und umständlich und/oder die Bewertung des Sterilisationsverfahrens sehr zeitaufwendig.

Der Erfindung lag die Aufgabe zugrunde, einen Bioindikator zur mikrobiologischen Kontrolle von Gassterilisationsverfahren zu schaffen, der die Nachteile der bisher bekannten Bioindikatoren nicht aufweist, der preisgünstig herzustellen, gut zu standardisieren, einfach und sicher anzuwenden sowie schnell und problemlos auszuwerten ist.

Gelöst wurde diese aufgabe durch einen Bioindikator zur mikrobiologischen Kontrolle von Gassterilisationsverfahren, der Sporenträger und ggf. Nährmediumbehälter in einer speziellen Tiefziehpackung enthält.

Gegenstand der Erfindung ist ein Bioindikator zur mikrobiologischen Kontrolle von Gassterilisationsverfahren, bestehend im wesentlichen aus einem Sporenträger (1) aus angeätzten oder angeschliffenen Glaskugeln oder -ringen oder aus entsprechenden Formteilen aus Keramik oder Kunststoff mit rauher Oberfläche in einem mit einer gas- und wasserdampfdurchlässigen Folie (3,9) verschlossenen Behälter (2), dadurch gekennzeichnet, daß dieser als Tiefziehpackung gestaltet ist und einen derart angeordneten, abgeschlossenen Nährmediumbehälter (6) enthält, daß der Sporenträger (1) in den mit einer durchdrückbaren Folie (7) verschlossenen Nährmediumbehälter (6) gedrückt werden kann.

Der Bioindikator kann auch so gestaltet sein, daß auf dem mit einer eindrückbaren Folie (7) verschlossenen Nährmediumbehälter (6) eine als Behälter für den Sporenträger (1) dienende Hülse (10) angeordnet ist, die mit einer gas- und wasserdampfdurchlässigen Folie (9) verschlossen ist. Es können auch mehrere Sporenträger (1) mit gegebenenfalls unterschiedlichem Sporengehalt bzw. paarweise Sporenträger (1) und Nährmediumbehälter (6) in mit einer gas- und wasserdampfdurchlässigen Folie (3, 9) verschlossenen Behältern (2) angeordnet sein.

Die Bioindikatoren enthalten einen oder mehrere Sporenträger (1), die aus angeätzten oder angeschliffenen Glaskugeln oder -ringen oder aus entsprechenden Formteilen aus Keramik oder Kunststoff mit rauher Oberfläche bestehen. Die Glaskugeln weisen zweckmäßigerweise einen Durchmesser von etwa 2 - 8 mm, vorzugsweise etwa 5 mm auf. Keramikringe sollten einen Durchmesser von etwa 4 - 8 mm, vorzugsweise etwa 6 mm, besitzen. Die Sporenträger werden z.B. mit $10^3$, $10^5$ bzw. $10^7$ Sporen/Träger beladen. Als Indikatormikroorganismen für Bioindikatoren zur mikrobiologischen Kontrolle von Gassterilisationsverfahren werden beispielsweise Sporen von Bacillus subtilis var. niger verwendet. Im gleichen Sinne können Sporen anderer sporenbildender Bakterien eingesetzt werden. Die Sporen werden aus ethanolischen oder wäßrigen Suspensionen auf die Formteile aufgebracht, wobei die Suspensionen Salze, Indikatorfarbstoffe, Gelatine, verschiedene Cellulosederivate, Polyvinylalkohole oder Polyvinylpyrrolidone enthalten können.

Als gas- und wasserdampfdurchlässige Folien (3, 9) zum Verschluß des Behälters mit dem Sporenträger (1) können alle Folienmaterialien oder Vliese verwendet werden, die eine gute Durchlässigkeit für gasförmige chemische Sterilisationsagenzien besitzen, die wasserdampfdurchlässig und zumindest im trockenen Zustand bakteriendicht sind sowie auf Kunststoffe wie Polyethylen, Polypropylen, Polyvinylchlorid oder entsprechende Mischpolymerisate aufgesiegelt werden können. Gut geeignet sind z.B. Sterilisationspapier nach DIN 58953 oder spezielle Vliese aus Polyethylenfasern.

Der transparente Nährmediumbehälter (6) besteht aus Glas oder Kunststoffen wie Polyethylen oder Polypropylen, auf den eine leicht zu durchstoßende dünne Folie (7) mit äußerst geringer Gas- und Wasserdampfdurchlässigkeit aufgesiegelt ist. Etwa 10 - 15 μm dicke Aluminiumfolie, die mit einem Siegellack beschichtet ist, eignet sich vorzugsweise als leicht durchstoßbare Folie (7) zum Verschluß eines Nährmediumbehälters aus Polypropylen oder Polyethylen.

In der Druckschrift DE-OS 14 92 498 ist ein wasserdurchlässiger Behälter mit trockenem Nährmedium und einem saugfähigen Papier als Träger für die Mikroorganismen beschrieben. Der behälter wird während der Prüfzeitspanne in Wasser getaucht. Dem Bioindikator nach der Erfindung liegt ein völlig anderes Konzept zugrunde: der Behälter soll gerade nicht wasserdurchlässig sein; das Nährmedium ist flüssig; die Mikroorganismen liegen nicht auf einem saugfähigen Träger vor; das Eintauchen in Wasser während der Inkubationszeit wäre sinnlos.

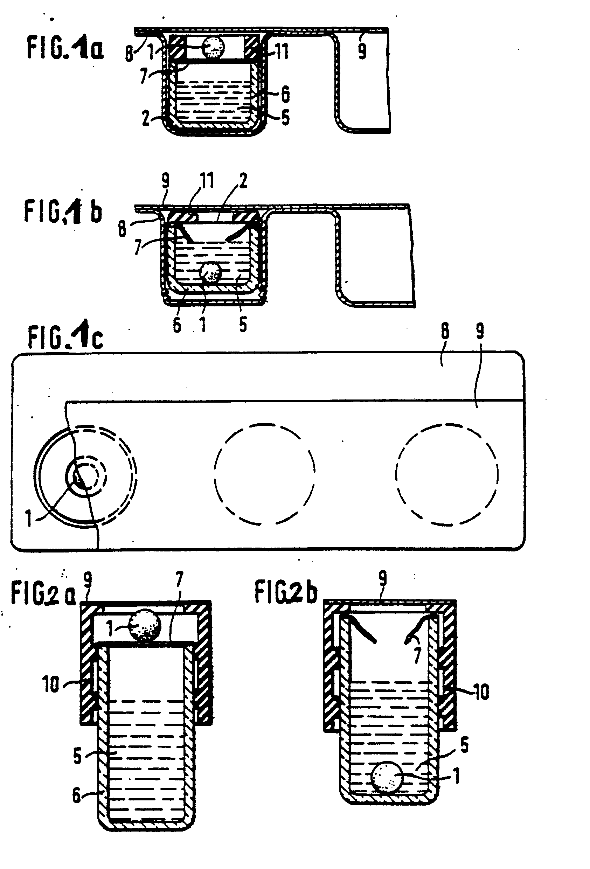

Die Erfindung wird anhand der Abbildungen 1 und 2 näher erläutert:

Der Bioindikator nach Fig. 1 und 2 enthält den Sporenträger (1) und ein Behältnis mit Nährmedium(6) in einem Tiefziehteil (8). Der Nährmediumbehälter (6) mit dem Nährmedium (5) ist aus transparentem Material gefertigt, damit von außen das Bakterienwachstum durch Trübung und/oder Farbveränderung etwa von pH-Indikatoren verfolgt werden kann. Der Nährmediumbehälter (6) ist mit einer eindrückbaren Folie (7) gasdicht verschlossen.

Fig. 1 a zeigt eine Ausführungsform des Bioindikators im Schnitt, bei der mehrere Sporenträger (1) und Nährmediumbehälter (6) paarweise in Vertiefungen (2) eines Tiefziehteiles (8) angeordnet sind. Auf das Tiefziehteil (8) ist eine gas- und wasserdampfdurchlässige Folie (9) aufgesiegelt.

Über dem transparenten Nährmediumbehälter (6), der mit einer dünnen, leicht zu durchstoßenden Folie (7) gas- und wasserdampfdicht verschlossen ist, befindet sich der Sporenträger (1). Dieser kann beispielsweise durch einen flexiblen Kunststoffring (11) zentral über dem Nährmediumbehälter gehalten werden. Zur Beurteilung des Sterilisationserfolges wird durch Eindrücken des flexiblen Tiefziehteilbodens im Bereich des Nährmediumbehälters (6) die dünne Verschlußfolie (7) des Nährmediumbehälters gegen den Sporenträger (1) gepreßt, so daß die Folie (7), wie in Fig. 1 b dargestellt, einreißt und der Sporenträger in das Nährmedium (5) gelangt.

Fig. 1 c zeigt das Bioindikator-Set im Aufriß.

Fig. 2 zeigt eine Ausführungsform des Bioindikators, bei der auf einen mit einer gas- und wasserdampfdichtem eindrückbaren Folie (7) verschlossenen Nährmediumbehälter (6) eine Hülse (10) aufgesteckt wird, die als Behälter für den Sporenträger (1) dient und die mit einer gas- und wasserdampfdurchlässigen Folie (9) verschlossen ist. Durch Zusammenschieben von Nährmediumbehälter (6) und aufgesteckter Hülse (10) und somit Zerreißen der dünnen Folie (7) kann der Sporenträger (1) in das Nährmedium (5) befördert werden. Die Folie (9) muß reißfest sein.

Zur mikrobiologischen Kontrolle von Gassterilisationsverfahren wird der Bioindikator mit dem zu sterilisierenden Gut in die Sterilisationskammer gegeben. Nach der Exposition der Bioindikatoren in der Sterilisationskammer und Überführung der Sporenträger in ein Nährmedium werden die Nährmediumbehälter mit den Sporenträgern z.B. 24 h bei 35° C bebrütet. Bei einem Wachstum der Testkeime wird z.B. ein Glucose enthaltendes Nährmedium durch Abbau von Glukose angesäuert, was den Farbumschlag eines pH-Indikators bewirkt. Das Wachstum ist an der Trübung des Nährmediums zu erkennen.

Da bei chemischen Sterilisationsverfahren einzelne Keime innerhalb einer großen Population häufig unter den normalen Sterilisationsbedingungen nicht abgetötet, sondern nur stark geschädigt werden, erscheint eine generelle Bewertung des Sterilisationsverfahrens nach der Endpunkt-Methode, d.h. einer Überprüfung auf Sterilität "Ja/Nein", infolge einer zu langen Analysenzeit für die Routine nicht empfehlenswert.

Bei einer Bewertung des Sterilisationsverfahrens durch Auswertung des Keimwachstums von Trägern mit z.B. $10^3$, $10^5$ bzw. $10^7$ Sporen nach einer Bebrütungszeit von 24 Stunden gewinnt man sichere Anhaltspunkte dafür, wie effizient das Sterilisationsverfahren war. Zeigen alle 3 Trägertypen nach 24 Stunden Inkubation in einem Testnährmedium kein Wachstum, so kann man davon ausgehen, daß unter den gewählten Sterilisationsbedingungen mindestens $10^6$ Keime pro Träger abgetötet wurden.

Durch eine längere Bebrütungszeit läßt sich diese Aussage zwar noch etwas präzisieren, jedoch ist für Routineuntersuchungen eine Bebrütungszeit von 24 Stunden voll ausreichend, wenn der Keimgehalt/Testfläche auf dem zu sterilisierenden Gut deutlich geringer ist als auf dem Sporenträger mit der mittleren Sporenkonzentration von $10^5$ Sporen/Träger, so daß eine ausreichende Sterilisationssicherheit vorhanden ist. Die Bebrütung erfolgt in der Regel bei 35° C. Als Nährmedium wird vorzugsweise ein Nährmedium mit Pepton, Glucose und einem pH-Indikator verwendet. Die Behälter mit dem Nährmedium werden jeweils vor der Fertigung der Bioindikatoren-Sets, z.B. durch γ-Bestrahlung, sterilisiert.

**Beispiel 1**

Als Sporenträger werden oberflächlich rauh geschliffene Glaskugeln vom Durchmesser 5,0 ± 0,1 mm verwendet. Die Sporen von Bacillus subtilis var. niger werden aus einer ethanolischen Suspension auf die Träger gebracht. Die mit $10^3$, $10^5$ und $10^7$ Sporen pro Träger beladenen Sporenträger werden einzeln in etwa 7 mm breite und 7 mm tiefe Behälter eines Tiefziehteils aus einer 250 μm dicken Polyethylenfolie gegeben. Auf das Tiefziehteil wird ein abziehbares Sterilisationspapier nach DIN 58953 (100 g/m²) aufgesiegelt. Nach der Exposition der Bioindikatoren in der Sterilisationskammer wird die abziehbare Deckfolie über dem Sporenträger-Behälter abgezogen und der Sporenträger in einen Behälter mit Nährmedium aus 5,0 g Pepton, 5,0 g Glucose, 30 mg/l Bromthymolblau (pH 7,3 ± 0,05) überführt. Nach 24 Stunden Inkubation bei 35° C wird das Nährmedium auf Keimwachstum untersucht, das an einem Umschlag der pH-Indikatorfarbe von Blau nach Gelb zu erkennen ist.

**Beispiel 2**

Als Sporenträger werden oberflächlich rauh geschliffene Glaskugeln vom Durchmesser 5,0 ± 0,1 mm verwendet. Die Sporen von Bacillus subtilis var. niger werden aus einer Suspension in 1-molarer Natriumchloridlösung, die 5 g Bromthymolblau pro Liter enthält, auf die Träger aufgebracht. Jeweils 3 Sporenträger mit $10^3$, $10^5$ und $10^7$ Sporen/Träger werden für ein Bioindikator-Set verwendet.

Die Nährmediumbehälter aus einem Polyethylen-Spritzgußteil mit 1,5 cm Durchmesser und 1,2 cm Höhe sind zu 2/3 mit Nährmedium gefüllt. Auf die Nährmediumbehälter ist eine 10 µm dicke Aluminiumfolie, die mit einem Siegellack beschichtet ist, aufgesiegelt. Der Behälter wird bei 28 K Gray ( ± 10 %) γ-strahlensterilisiert. Die sterilen Nährmediumbehälter werden in Vertiefungen eines Tiefziehteils aus einer 300 µm dicken Polyethylenfolie eingesetzt. Auf die Nährmediumbehälterdeckel wird ein Schaumstoffring mit einem Innendurchmesser von etwa 7 mm und einer Höhe von etwa 5 mm aufgesetzt. Jeweils ein Sporenträger wird in die Ringöffnung gesetzt. Auf das Tiefziehteil mit Nährmediumbehälter und Sporenträger wird ein Sterilisationspapier nach DIN 58953 (100 g/m²) aufgesiegelt.

Nach der Exposition des Bioindikators in der Sterilisationskammer wird der Nährmediumbehälter durch Druck auf das leicht verformbare Tiefziehteil so gegen den Sporenträger gepreßt, daß die Aluminiumfolie des Nährmediumbehälters reißt und der Sporenträger in das Nährmedium gelangt. Das Bioindikator-Set wird 24 Stunden bei 35° C bebrütet. Eine Verfärbung des Nährmediums nach Gelb zeigt an, daß unter den gewählten Sterilisationsbedingungen nicht alle Sporen von Bacillus subtilis var. niger abgetötet wurden.

**Beispiel 3**

Als Sporenträger werden Keramikringe mit rauher Oberfläche von 6,0 ± 0,1 mm Außendurchmesser, 3,0 ± 0,1 mm Innendurchmesser und 5,0 ± 0,1 mm Höhe verwendet. Die Träger werden mit $10^3$, $10^5$ bzw. $10^7$ Sporen beladen.

Zylinderförmige durchsichtige Kunststoffbehälter (Spritzgußteile aus Polypropylen) von 1,2 cm Durchmesser und 3 cm Höhe werden zu 2/3 mit Nährmedium gefüllt, mit einer 15 µm dicken siegellackbeschichteten Aluminiumfolie dicht verschlossen und bei 28 K

Gray ( ± 10 %) γ-strahlensterilisiert. Der Nährmediumbehälter wird in eine Polypropylenhülse mit einem Sporenträger bis zu einem Anschlag etwa in der Mitte der Hülse gesteckt. Auf die Hülse ist an ihrer Oberseite ein gas- und wasserdampfdurchlässiges Vlies aus Polyethylenfasern (Tyvek®) (70 g/m²) aufgesiegelt.

Nach der Exposition des Bioindikators in der Sterilisationskammer wird durch ein Zusammenschieben von Nährmediumbehälter und Hülse der Sporenträger durch die dabei aufreißende Aluminiumfolie in das Nährmedium befördert. Nach 24 Stunden Inkubation bei 35° C wird festgestellt, ob Wachstum von Bacillus subtilis var. niger an einem Umschlag des pH-Indikators von Blau nach Gelb zu erkennen ist.

**Patentansprüche**

1. Bioindikator zur mikrobiologischen Kontrolle von Gassterilisationsverfahren, bestehend im wesentlichen aus einem Sporenträger (1) aus angeätzten oder angeschliffenen Glaskugeln oder -ringen oder aus entsprechenden Formteilen aus Keramik oder Kunststoff mit rauher Oberfläche in einem mit einer gas- und wasserdampfdurchlässigen Folie (3, 9) verschlossenen Behälter (2), dadurch gekennzeichnet, daß dieser als Tiefziehpackung gestaltet ist und einen derart angeordneten, abgeschlossenen Nährmediumbehälter (6) enthält, daß der Sporenträger (1) in den mit einer durchdrückbaren Folie (7) verschlossenen Nährmediumbehälter (6) gedrückt werden kann.

2. Bioindikator nach Anspruch 1, dadurch gekennzeichnet, daß auf dem mit einer eindrückbaren Folie (7) verschlossenen Nährmediumbehälter (6) eine als Behälter für den Sporenträger (1) dienende Hülse (10) angeordnet ist, die mit einer gas- und wasserdampfdurchlässigen Folie (9) verschlossen ist.

3. Bioindikator nach den Ansprüchen 1 und 2, dadurch gekennzeichnet, daß mehrere Sporenträger (1) mit gegebenenfalls unterschiedlichem Sporengehalt bzw. paarweise Sporenträger (1) und Nährmediumbehälter (6) in mit einer gas- und wasserdampfdurchlässigen Folie (3, 9) verschlossenen Behältern (2) angeordnet sind.

**Claims**

1. Bioindicator far monitoring gas sterilisation processes by microbiological means, consisting essentially of a spore carrier (1) of etched or ground glass spheres or rings or of corresponding ceramic or plastic-mouldings having a rough surface in a container (2), closed by a film (3, 9) which is permeable to gas and water vapour characterised in that the latter is in the form of a thermoformed package in which the nutrient medium container (6) is arranged in such a way that the spore carrier (1) can be forced into the nutrient medium container (6) which is closed by a press-through film (7).

2. Bioindicator according to Claim 1, characterised in that a tube (10) which serves as a container for the spore carrier (1) and which is closed by a film (9), permeable to gas and water vapour, is mounted on the nutrient medium container (6) which is closed by a crushable film (7).

3. Bioindicator according to Claims 1 and 2, characterised in that several spore carriers (1) having, if appropriate, different spore contents, or spore carriers (1) and nutrient medium containers (6) in pairs are located in containers (2) which are closed by a film (3, 9) which is permeable to gas and water vapour.

## Revendications

1. Bio-indicateur pour le contrôle microbiologique des procédés de stérilisation par le gaz, constitué essentiellement d'un support de spores (1) en billes ou bagues de verre décapées ou meulées ou de pièces de forme analogues en céramique ou en matière plastique à surface rugueuse dans un récipient (2) fermé par une feuille (3, 9) perméable aux gaz et à la vapeur d'eau, caractérisé en ce que ce récipient est constitué par un emballage embouti et contient un récipient à milieu nutritif (6) fermé et disposé de façon à ce que le support de spores (1) puisse être enfoncé dans le récipient à milieu nutritif (6) fermé par une feuille (7) pouvant être défoncée.

2. Bio-indicateur selon la revendication 1, caractérisé en ce que, sur le récipient à milieu nutritif (6) fermé par une feuille (7) pouvant être défoncée, est appliquée une douille (10) servant de récipient pour le support de spores (1), qui est fermée par une feuille (9) perméable aux gaz et à la vapeur d'eau.

3. Bio-indicateur selon les revendications 1 et 2, caractérisé en ce que plusieurs supports de spores (1) avec, éventuellement, des teneurs en spores différentes ou des supports de spores (1) et des récipients à milieu nutritif (6) disposés par paires se trouvent dans des récipients (2) fermés par une feuille (3, 9) perméable aux gaz et à la vapeur d'eau.

FIG.1a

FIG.1b

FIG.1c

FIG.2a

FIG.2b